# EUROPEAN PATENT APPLICATION

(11) **EP 3 861 984 A1**
(43) Date of publication of application: **11.08.2021**
(21) Application number: 21155478.7
(22) Date of filing: 05.02.2021
(51) Int. Cl.: A61K 9/00, A61K 9/12, A61K 47/10, A61L 2/20

(54) **OZONE FOAM FOR TREATING DERMATITIS**

(30) Priority: 07.02.2020 US 202062971646 P
(71) Applicant: OP-Hygiene IP GmbH, 4704 Niederbipp (CH)
(72) Inventor: OPHARDT, Heiner, 4422 Arisdorf (CH)
(74) Representative: Grünecker Patent- und Rechtsanwälte PartG mbB

(57) **Abstract**

A method of placing a glove onto a user's hand. In a first step, the user's hand is coated in foam. In a second step, the user's hand is inserted into the glove, while the user's hand is coated in the foam. The foam may contain ozone in a concentration that is selected to treat and/or prevent dermatitis. The foam may also contain alcohol for disinfecting the user's hand.

## Description

### Field of the Invention

This invention relates to hand cleaning foam dispensers, and more particularly to dispensers that dispense ozone containing foam.

### Background of the Invention

Dispensers that dispense ozone containing foam for sanitizing a user's hands are known in the art. For example, United States Patent No. 8,733,596 to Ophardt et al., issued May 27, 2014, which is incorporated herein by reference, teaches the use of ultraviolet radiation or corona discharges to generate ozone from the oxygen in air. The resulting ozonated air is then passed through a foam generator together with a suitable foamable liquid to generate the ozone containing foam, which is dispensed onto a user's hands to provide a sanitizing effect.

It is also known that low doses of ozone, in the form of ozonated saline, can promote wound healing, as is described in Valacchi et al., "Ozone mediators effect on 'in vitro' scratch wound closure" Free Radic Res. 2016 Sep;50(9): 1022-31, which is incorporated herein by reference. The therapeutic activity of ozone is also described in Zanardi et al., "Ozone: A Multifaceted Molecule with Unexpected Therapeutic Activity" Curr Med Chem. 2016;23(4):304-14, which is incorporated herein by reference. A low dose of ozone is believed to induce a transient and mild oxidative stress, which activates cellular defense mechanisms that can have a protective and healing effect.

The inventors of the present invention have appreciated that known hand hygiene protocols that require frequent hand washing with soap, and the prolonged use of gloves, can increase the risk of developing hand eczema or dermatitis. In addition, washing or disinfecting hands with soap and/or an alcohol based hand sanitizer before putting on gloves, as is often required by hand hygiene protocols, removes oils from the hands. This can make it more difficult to put on gloves and potentially increases skin irritation. As a result, many workers do not wash and/or disinfect their hands prior to putting on gloves, which increases the risk of disease transmission.

The inventors have also appreciated that known methods of generating and using ozone containing foam suffer a number of disadvantages. For example, known dispensers generally only provide ozone containing foam for the limited purpose of killing pathogens on a user's hands.

### Summary of the Invention

To at least partially overcome some of the disadvantages of previously known methods and devices, in a first aspect the present invention provides a method of treating and/or preventing dermatitis on a user's hand by applying an ozone containing foam to the user's hand. The foam preferably includes a low dose of ozone that is selected to treat and/or prevent dermatitis on the user's hand, and may, for example, be selected to stimulate the body's natural defense mechanisms and repair mechanisms.

The inventors have appreciated that an ozone containing foam is a particularly well suited medium by which to deliver a low dose of ozone to a user's hand, for the purpose of treating or preventing dermatitis. In particular, an ozone containing foam in which bubbles of an ozone containing gas are suspended in a foamable liquid can be distributed evenly over a user's hand with relative ease, while requiring only a relatively small volume of the foamable liquid. In addition, an ozone containing foam can be generated relatively quickly and conveniently by converting the oxygen in air into ozone to produce ozonated air, and then delivering the ozonated air through a foam generator together with a foamable liquid to generate the ozone containing foam.

Furthermore, an ozone containing foam is well suited to providing other functions in addition to treating and/or preventing dermatitis. The ozone containing foam may, for example, contain a disinfecting agent, such as an alcohol like ethanol and/or isopropanol, that disinfects or sanitizes the user's hands. The ozone in the foam may also act as a disinfecting agent that disinfects or sanitizes the user's hands. As the risk of developing dermatitis, or aggravating an existing case of dermatitis, may act as a disincentive for proper hand hygiene compliance, providing a hand sanitizing foam that is also formulated to protect against and/or treat dermatitis may help to reduce, remove, and/or reverse this disincentive.

In another aspect, the present invention provides a method of placing a glove onto a user's hand by first coating the user's hand in foam, and then sliding the user's hand, while the user's hand is coated in the foam, into the glove. The inventors have appreciated that having foam on the user's hand can reduce friction between the hand and the glove, making it easier to put on the glove and potentially reducing skin irritation. As a result, compliance with hand hygiene protocols that require disinfecting the user's hands before putting on gloves may be improved. Preferably, the foam that is used to coat the user's hand before putting on the glove also disinfects the user's hand, so that the foam provides both a disinfecting and a friction reducing function. The foam may, for example, contain alcohol such as ethanol and/or isopropanol as a disinfecting agent. The foam also preferably includes a low dose of ozone for treating and/or preventing dermatitis.

In a further aspect, the present invention provides a dispenser for dispensing an ozone containing foam, including an ozone adjustor for adjusting an amount of ozone that is dispensed in the ozone containing foam upon activation of the dispenser. The inventors have appreciated that providing an ozone adjustor allows the dispenser to be used to dispense foams having different concentrations of ozone for different purposes. For example, the ozone adjustor could be used to select a first amount of ozone that is suitable for treating dermatitis, or a second amount of ozone that is suitable for killing pathogens on the user's hands. Different users having different needs could thus select the amount of ozone that is suitable for their specific needs. The ozone adjustor could also, for example, be used to select an ozone-free mode of operation in which the dispenser dispenses a foam that is ozone-free.

Accordingly, in one aspect the present invention resides in a method of placing a glove onto a user's hand comprising:
coating the user's hand in foam; and
sliding the user's hand, while the user's hand is coated in the foam, into the glove.

Preferably, the foam contains ozone.

The foam may, for example, have a concentration of ozone that is selected to treat dermatitis on the user's hand; have a concentration of ozone that is selected to protect the user's hand from dermatitis; have a concentration of ozone that is selected to promote healing of the user's hand; and/or have a concentration of ozone that is selected to kill pathogens on the user's hand.

Preferably, the foam disinfects the user's hand.

The foam may, for example, contain alcohol for disinfecting the user's hand.

Preferably, the foam is selected to remain stable for a preselected period of time on the user's hand.

The foam may, for example, comprise bubbles of gas in a foamable liquid, wherein at least one of the gas, the foamable liquid, a size of the bubbles, a ratio of the gas to the foamable liquid, and a method of generating the foam are selected so that at least some of the bubbles remain intact for the preselected period of time on the user's hand.

Optionally, at least 50% of the bubbles of gas remain intact for at least 30 seconds on the user's hand.

In another aspect, the present invention resides in a method of treating dermatitis on a user's hand comprising: applying an ozone containing foam to the user's hand.

In a further aspect, the present invention resides in a method of preventing dermatitis on a user's hand comprising: applying an ozone containing foam to the user's hand.

In a further aspect, the present invention resides in a method of treating dermatitis on a user's hand comprising: placing the user's hand and an ozone containing foam into a glove.

In a further aspect, the present invention resides in a method of preventing dermatitis on a user's hand comprising: placing the user's hand and an ozone containing foam into a glove.

In a further aspect, the present invention resides in use of an ozone containing foam to treat dermatitis on a user's hand.

In a further aspect, the present invention resides in use of an ozone containing foam to prevent dermatitis on a user's hand.

In a further aspect, the present invention resides in use of a foam to assist in sliding a user's hand into a glove.

Preferably, the foam contains ozone.

The foam may, for example, have a concentration of ozone that is selected to treat dermatitis on the user's hand; have a concentration of ozone that is selected to protect the user's hand from dermatitis; have a concentration of ozone that is selected to promote healing of the user's hand; and/or have a concentration of ozone that is selected to kill pathogens on the user's hand.

Preferably, the foam disinfects the user's hand.

The foam may, for example, contain alcohol for disinfecting the user's hand.

Preferably, the foam is selected to remain stable for a preselected period of time on the user's hand.

The foam may, for example, comprise bubbles of gas in a foamable liquid, wherein at least one of the gas, the foamable liquid, a size of the bubbles, a ratio of the gas to the foamable liquid, and a method of generating the foam are selected so that at least some of the bubbles remain intact for the preselected period of time on the user's hand.

Optionally, the foam is applied to the user's hand and/or into an inner cavity of the glove.

In another aspect, the present invention resides in a method of placing a glove onto a user's hand comprising:
applying foam into an inner cavity of the glove; and
sliding the user's hand into the glove.

Preferably, the foam contains ozone.

In a further aspect, the present invention resides in a dispenser for dispensing an ozone containing foam, the dispenser comprising:
an ozone generator that generates an ozone containing gas;
a foam generator that receives the ozone containing gas and mixes the ozone containing gas with a foamable liquid to generate the ozone containing foam;
a discharge outlet for discharging the ozone containing foam; and
an ozone adjustor for adjusting an amount of ozone that is dispensed in the ozone containing foam upon activation of the dispenser.

Optionally, the ozone adjustor adjusts at least one of: a concentration of ozone in the ozone containing gas generated by the ozone generator; a volume of the ozone containing gas that is mixed with the foamable liquid by the foam generator; a ratio of the ozone containing gas to the foamable liquid; and a volume of the ozone containing foam that is dispensed from the discharge outlet.

In some embodiments, the ozone adjustor allows for selection of the amount of ozone that is dispensed in the ozone containing foam at least from:
a first amount of ozone that is selected to treat dermatitis; and
a second amount of ozone that is selected to kill pathogens.

The ozone adjustor may, for example, allow for selection of an ozone-free mode of operation, wherein an ozone-free fluid is dispensed from the dispenser.

Optionally, when in the ozone-free mode of operation, the foam generator receives an ozone-free gas and mixes the ozone-free gas with the foamable liquid to generate an ozone-free foam; and the ozone-free foam is discharged from the discharge outlet.

In some embodiments, the dispenser further comprises:
a reservoir containing a supply of the foamable liquid;
a liquid pump for delivering the foamable liquid to the foam generator; and
a gas pump for delivering the ozone containing gas to the foam generator.

Optionally, the dispenser further comprises a sensor that senses a concentration of ozone in the ozone containing gas, or that senses a parameter for estimating or calculating the concentration of ozone in the ozone containing gas.

The dispenser may also comprise a controller that controls the ozone generator so that the concentration of ozone in the ozone containing gas is within a preselected range of concentrations.

The foamable liquid is preferably a hand disinfectant.

In a first aspect the present invention resides in use of a foam to assist in sliding a hand into a glove.

In a second aspect the present invention resides in the use of a foam, which optionally incorporates one or more features of the first aspect, wherein the foam contains ozone.

In a third aspect the present invention resides in the use of a foam, which optionally incorporates one or more features of one or more of the first and second aspects, wherein the foam has a concentration of ozone that is selected to treat dermatitis on the hand.

In a fourth aspect the present invention resides in the use of a foam, which optionally incorporates one or more features of one or more of the first to third aspects, wherein the foam has a concentration of ozone that is selected to protect the hand from dermatitis.

In a fifth aspect the present invention resides in the use of a foam, which optionally incorporates one or more features of one or more of the first to fourth aspects, wherein the foam has a concentration of ozone that is selected to promote healing of the hand.

In a sixth aspect the present invention resides in the use of a foam, which optionally incorporates one or more features of one or more of the first to fifth aspects, wherein the foam has a concentration of ozone that is selected to kill pathogens on the hand.

In a seventh aspect the present invention resides in the use of a foam, which optionally incorporates one or more features of one or more of the first to sixth aspects, wherein the foam disinfects the hand.

In an eighth aspect the present invention resides in the use of a foam, which optionally incorporates one or more features of one or more of the first to seventh aspects, wherein the foam contains alcohol for disinfecting the hand.

In a ninth aspect the present invention resides in the use of a foam, which optionally incorporates one or more features of one or more of the first to eighth aspects, wherein the foam comprises bubbles of gas in a foamable liquid.

In a tenth aspect the present invention resides in the use of a foam, which optionally incorporates one or more features of one or more of the first to ninth aspects, wherein the foam is selected to remain stable for a preselected period of time on the hand.

In an eleventh aspect the present invention resides in the use of a foam, which optionally incorporates one or more features of one or more of the first to tenth aspects, wherein at least one of the gas, the foamable liquid, a size of the bubbles, a ratio of the foamable liquid to the gas, and a method of generating the foam are selected so that at least some of the bubbles remain intact for the preselected period of time on the hand.

In a twelfth aspect the present invention resides in the use of a foam, which optionally incorporates one or more features of one or more of the first to eleventh aspects, wherein at least 50% of the bubbles of gas remain intact for at least 30 seconds on the hand.

In a thirteenth aspect the present invention resides in the use of a foam, which optionally incorporates one or more features of one or more of the first to twelfth aspects, wherein the foamable liquid is a hand disinfectant.

In a fourteenth aspect the present invention resides in the use of a foam, which optionally incorporates one or more features of one or more of the first to thirteenth aspects, wherein the bubbles are smaller than 2 mm.

In a fifteenth aspect the present invention resides in the use of a foam, which optionally incorporates one or more features of one or more of the first to fourteenth aspects, wherein the bubbles are smaller than 1 mm.

In a sixteenth aspect the present invention resides in the use of a foam, which optionally incorporates one or more features of one or more of the first to fifteenth aspects, wherein the bubbles have a mean bubble diameter in a range of 50 µm to 600 µm.

In a seventeenth aspect the present invention resides in the use of a foam, which optionally incorporates one or more features of one or more of the first to sixteenth aspects, wherein the bubbles have a mean bubble diameter in a range of 100 µm to 300 µm.

In an eighteenth aspect the present invention resides in the use of a foam, which optionally incorporates one or more features of one or more of the first to seventeenth aspects, wherein the foam has a ratio of about 1:5 of the foamable liquid to the gas by volume.

In a nineteenth aspect the present invention resides in the use of a foam, which optionally incorporates one or more features of one or more of the first to eighteenth aspects, wherein the foam has a ratio of about 1:10 of the foamable liquid to the gas by volume.

In a twentieth aspect the present invention resides in the use of a foam, which optionally incorporates one or more features of one or more of the first to nineteenth aspects, wherein the foam has a ratio of about 1:15 of the foamable liquid to the gas by volume.

In a twenty first aspect the present invention resides in the use of a foam, which optionally incorporates one or more features of one or more of the first to twentieth aspects, wherein the foam has a ratio of about 1:50 of the foamable liquid to the gas by volume.

In a twenty second aspect the present invention resides in the use of a foam, which optionally incorporates one or more features of one or more of the first to twenty first aspects, wherein the foam has a ratio of between about 1:5 and about 1:60 of the foamable liquid to the gas by volume.

In a twenty third aspect the present invention resides in the use of a foam, which optionally incorporates one or more features of one or more of the first to twenty second aspects, wherein the foam comprises about 0.5 mL of the foamable liquid and about 2.5 mL of the gas.

In a twenty fourth aspect the present invention resides in the use of a foam, which optionally incorporates one or more features of one or more of the first to twenty third aspects, wherein the foam comprises about 1 mL of the foamable liquid and about 5 mL of the gas.

In a twenty fifth aspect the present invention resides in the use of a foam, which optionally incorporates one or more features of one or more of the first to twenty fourth aspects, wherein the foam comprises about 1 mL of the foamable liquid and about 10 mL of the gas.

In a twenty sixth aspect the present invention resides in the use of a foam, which optionally incorporates one or more features of one or more of the first to twenty fifth aspects, wherein the foam comprises between about 0.5 mL and 10 mL of the foamable liquid and between about 2.5 mL and 100 mL of the gas.

In a twenty seventh aspect the present invention resides in the use of a foam, which optionally incorporates one or more features of one or more of the first to twenty sixth aspects, wherein the foamable liquid comprises at least one of: ethanol, isopropanol, n-propanol, and mixtures thereof.

In a twenty eighth aspect the present invention resides in the use of a foam, which optionally incorporates one or more features of one or more of the first to twenty seventh aspects, wherein the foamable liquid comprises about 20 to 90% alcohol by volume and about 80 to 10% water by volume.

In a twenty ninth aspect the present invention resides in the use of a foam, which optionally incorporates one or more features of one or more of the first to twenty eighth aspects, wherein the foamable liquid comprises a foaming agent.

In a thirtieth aspect the present invention resides in the use of a foam, which optionally incorporates one or more features of one or more of the first to twenty ninth aspects, wherein the foaming agent comprises a surfactant.

In a thirty first aspect the present invention resides in the use of a foam, which optionally incorporates one or more features of one or more of the first to thirtieth aspects, wherein the foamable liquid comprises about 0.1% to 15% of the surfactant by volume.

In a thirty second aspect the present invention resides in the use of a foam, which optionally incorporates one or more features of one or more of the first to thirty first aspects, wherein the foam is applied to the hand.

In a thirty third aspect the present invention resides in the use of a foam, which optionally incorporates one or more features of one or more of the first to thirty second aspects, wherein the foam is applied into an inner cavity of the glove.

In a thirty fourth aspect the present invention resides in the use of a foam, which optionally incorporates one or more features of one or more of the first to thirty third aspects, wherein the foam contains 2 to 300 µM of ozone.

In a thirty fifth aspect the present invention resides in the use of a foam, which optionally incorporates one or more features of one or more of the first to thirty fourth aspects, wherein the foam contains 10 to 40 µg/mL of ozone.

In a thirty sixth aspect the present invention resides in the use of a foam, which optionally incorporates one or more features of one or more of the first to thirty fifth aspects, wherein the foam comprises an ozonated gas.

In a thirty seventh aspect the present invention resides in the use of a foam, which optionally incorporates one or more features of one or more of the first to thirty sixth aspects, wherein the ozonated gas has less than 0.05% ozone by volume.

In a thirty eighth aspect the present invention resides in the use of a foam, which optionally incorporates one or more features of one or more of the first to thirty seventh aspects, wherein the ozonated gas has less than 0.01% ozone by volume.

In a thirty ninth aspect the present invention resides in the use of a foam, which optionally incorporates one or more features of one or more of the first to thirty eighth aspects, wherein the ozonated gas has less than 0.005% ozone by volume.

In a fortieth aspect the present invention resides in the use of a foam, which optionally incorporates one or more features of one or more of the first to thirty ninth aspects, wherein the ozonated gas has less than 0.001% ozone by volume.

In a forty first aspect the present invention resides in the use of a foam, which optionally incorporates one or more features of one or more of the first to fortieth aspects, wherein the ozonated gas has less than 0.0001% ozone by volume.

In a forty second aspect the present invention resides in the use of a foam, which optionally incorporates one or more features of one or more of the first to forty first aspects, wherein the ozonated gas has less than 0.00001% ozone by volume.

In a forty third aspect the present invention resides in the use of a foam, which optionally incorporates one or more features of one or more of the first to forty second aspects, wherein the ozonated gas has less than 0.000001% ozone by volume.

In a forty fourth aspect the present invention resides in the use of a foam, which optionally incorporates one or more features of one or more of the first to forty third aspects, wherein the ozonated gas has between 0.000001% and 0.05% ozone by volume.

In a forty fifth aspect the present invention resides in the use of a foam, which optionally incorporates one or more features of one or more of the first to forty fourth aspects, wherein the ozonated gas comprises ozonated air.

In a forty sixth aspect the present invention resides in use of an ozone containing foam to treat dermatitis, which optionally incorporates one or more features of one or more of the first to forty fifth aspects.

In a forty seventh aspect the present invention resides in use of an ozone containing foam to prevent dermatitis, which optionally incorporates one or more features of one or more of the first to forty sixth aspects.

In a forty eighth aspect the present invention resides in the use of an ozone containing foam, which optionally incorporates one or more features of one or more of the first to forty seventh aspects, wherein the foam disinfects a user's hand.

In a forty ninth aspect the present invention resides in the use of an ozone containing foam, which optionally incorporates one or more features of one or more of the first to forty eighth aspects, wherein the foam contains alcohol for disinfecting the user's hand.

In a fiftieth aspect the present invention resides in the use of an ozone containing foam, which optionally incorporates one or more features of one or more of the first to forty ninth aspects, wherein the foam comprises bubbles of gas in a foamable liquid.

In a fifty first aspect the present invention resides in the use of an ozone containing foam, which optionally incorporates one or more features of one or more of the first to fiftieth aspects, wherein the foam is selected to remain stable for a preselected period of time.

In a fifty second aspect the present invention resides in the use of an ozone containing foam, which optionally incorporates one or more features of one or more of the first to fifty first aspects, wherein at least one of the gas, the foamable liquid, a size of the bubbles, a ratio of the foamable liquid to the gas, and a method of generating the foam are selected so that at least some of the bubbles remain intact for the preselected period of time.

In a fifty third aspect the present invention resides in the use of an ozone containing foam, which optionally incorporates one or more features of one or more of the first to fifty second aspects, wherein at least 50% of the bubbles of gas remain intact for at least 30 seconds.

In a fifty fourth aspect the present invention resides in the use of an ozone containing foam, which optionally incorporates one or more features of one or more of the first to fifty third aspects, wherein the foamable liquid is a hand disinfectant.

In a fifty fifth aspect the present invention resides in the use of an ozone containing foam, which optionally incorporates one or more features of one or more of the first to fifty fourth aspects, wherein the bubbles are smaller than 2 mm.

In a fifty sixth aspect the present invention resides in the use of an ozone containing foam, which optionally incorporates one or more features of one or more of the first to fifty fifth aspects, wherein the bubbles are smaller than 1 mm.

In a fifty seventh aspect the present invention resides in the use of an ozone containing foam, which optionally incorporates one or more features of one or more of the first to fifty sixth aspects, wherein the bubbles have a mean bubble diameter in a range of 50 µm to 600 µm.

In a fifty eighth aspect the present invention resides in the use of an ozone containing foam, which optionally incorporates one or more features of one or more of the first to fifty seventh aspects, wherein the bubbles have a mean bubble diameter in a range of 100 µm to 300 µm.

In a fifty ninth aspect the present invention resides in the use of an ozone containing foam, which optionally incorporates one or more features of one or more of the first to fifty eighth aspects, wherein the foam has a ratio of about 1:5 of the foamable liquid to the gas by volume.

In a sixtieth aspect the present invention resides in the use of an ozone containing foam, which optionally incorporates one or more features of one or more of the first to fifty ninth aspects, wherein the foam has a ratio of about 1:10 of the foamable liquid to the gas by volume.

In a sixty first aspect the present invention resides in the use of an ozone containing foam, which optionally incorporates one or more features of one or more of the first to sixtieth aspects, wherein the foam has a ratio of about 1:15 of the foamable liquid to the gas by volume.

In a sixty second aspect the present invention resides in the use of an ozone containing foam, which optionally incorporates one or more features of one or more of the first to sixty first aspects, wherein the foam has a ratio of about 1:50 of the foamable liquid to the gas by volume.

In a sixty third aspect the present invention resides in the use of an ozone containing foam, which optionally incorporates one or more features of one or more of the first to sixty second aspects, wherein the foam has a ratio of between about 1:5 and about 1:60 of the foamable liquid to the gas by volume.

In a sixty fourth aspect the present invention resides in the use of an ozone containing foam, which optionally incorporates one or more features of one or more of the first to sixty third aspects, wherein the foam comprises about 0.5 mL of the foamable liquid and about 2.5 mL of the gas.

In a sixty fifth aspect the present invention resides in the use of an ozone containing foam, which optionally incorporates one or more features of one or more of the first to sixty fourth aspects, wherein the foam comprises about 1 mL of the foamable liquid and about 5 mL of the gas.

In a sixty sixth aspect the present invention resides in the use of an ozone containing foam, which optionally incorporates one or more features of one or more of the first to sixty fifth aspects, wherein the foam comprises about 1 mL of the foamable liquid and about 10 mL of the gas.

In a sixty seventh aspect the present invention resides in the use of an ozone containing foam, which optionally incorporates one or more features of one or more of the first to sixty sixth aspects, wherein the foam comprises between about 0.5 mL and 10 mL of the foamable liquid and between about 2.5 mL and 100 mL of the gas.

In a sixty eighth aspect the present invention resides in the use of an ozone containing foam, which optionally incorporates one or more features of one or more of the first to sixty seventh aspects, wherein the foamable liquid comprises at least one of: ethanol, isopropanol, n-propanol, and mixtures thereof.

In a sixty ninth aspect the present invention resides in the use of an ozone containing foam, which optionally incorporates one or more features of one or more of the first to sixty eighth aspects, wherein the foamable liquid comprises about 20 to 90% alcohol by volume and about 80 to 10% water by volume.

In a seventieth aspect the present invention resides in the use of an ozone containing foam, which optionally incorporates one or more features of one or more of the first to sixty ninth aspects, wherein the foamable liquid comprises a foaming agent.

In a seventy first aspect the present invention resides in the use of an ozone containing foam, which optionally incorporates one or more features of one or more of the first to seventieth aspects, wherein the foaming agent comprises a surfactant.

In a seventy second aspect the present invention resides in the use of an ozone containing foam, which optionally incorporates one or more features of one or more of the first to seventy first aspects, wherein the foamable liquid comprises about 0.1% to 15% of the surfactant by volume.

In a seventy third aspect the present invention resides in the use of an ozone containing foam, which optionally incorporates one or more features of one or more of the first to seventy second aspects, wherein the foam is in an inner cavity of a glove.

In a seventy fourth aspect the present invention resides in the use of an ozone containing foam, which optionally incorporates one or more features of one or more of the first to seventy third aspects, wherein the foam contains 2 to 300 µM of ozone.

In a seventy fifth aspect the present invention resides in the use of an ozone containing foam, which optionally incorporates one or more features of one or more of the first to seventy fourth aspects, wherein the foam contains 10 to 40 µg/mL of ozone.

In a seventy sixth aspect the present invention resides in the use of an ozone containing foam, which optionally incorporates one or more features of one or more of the first to seventy fifth aspects, wherein the foam comprises an ozonated gas.

In a seventy seventh aspect the present invention resides in the use of an ozone containing foam, which optionally incorporates one or more features of one or more of the first to seventy sixth aspects, wherein the ozonated gas has less than 0.05% ozone by volume.

In a seventy eighth aspect the present invention resides in the use of an ozone containing foam, which optionally incorporates one or more features of one or more of the first to seventy seventh aspects, wherein the ozonated gas has less than 0.01% ozone by volume.

In a seventy ninth aspect the present invention resides in the use of an ozone containing foam, which optionally incorporates one or more features of one or more of the first to seventy eighth aspects, wherein the ozonated gas has less than 0.005% ozone by volume.

In an eightieth aspect the present invention resides in the use of an ozone containing foam, which optionally incorporates one or more features of one or more of the first to seventy ninth aspects, wherein the ozonated gas has less than 0.001% ozone by volume.

In an eighty first aspect the present invention resides in the use of an ozone containing foam, which optionally incorporates one or more features of one or more of the first to eightieth aspects, wherein the ozonated gas has less than 0.0001% ozone by volume.

In an eighty second aspect the present invention resides in the use of an ozone containing foam, which optionally incorporates one or more features of one or more of the first to eighty first aspects, wherein the ozonated gas has less than 0.00001% ozone by volume.

In an eighty third aspect the present invention resides in the use of an ozone containing foam, which optionally incorporates one or more features of one or more of the first to eighty second aspects, wherein the ozonated gas has less than 0.000001% ozone by volume.

In an eighty fourth aspect the present invention resides in the use of an ozone containing foam, which optionally incorporates one or more features of one or more of the first to eighty third aspects, wherein the ozonated gas has between 0.000001% and 0.05% ozone by volume.

In an eighty fifth aspect the present invention resides in the use of an ozone containing foam, which optionally incorporates one or more features of one or more of the first to eighty fourth aspects, wherein the ozonated gas comprises ozonated air.

In an eighty sixth aspect the present invention resides in a dispenser for dispensing an ozone containing foam, which optionally incorporates one or more features of one or more of the first to eighty fifth aspects, the dispenser comprising: an ozone generator that generates an ozone containing gas; a foam generator that receives the ozone containing gas and mixes the ozone containing gas with a foamable liquid to generate the ozone containing foam; and a discharge outlet for discharging the ozone containing foam; wherein the ozone containing foam is for at least one of: treating dermatitis and preventing dermatitis.

In an eighty seventh aspect the present invention resides in a dispenser for dispensing an ozone containing foam, which optionally incorporates one or more features of one or more of the first to eighty sixth aspects, the dispenser comprising: an ozone generator that generates an ozone containing gas; a foam generator that receives the ozone containing gas and mixes the ozone containing gas with a foamable liquid to generate the ozone containing foam; a discharge outlet for discharging the ozone containing foam; and an ozone adjustor for adjusting an amount of ozone that is dispensed in the ozone containing foam upon activation of the dispenser.

In an eighty eighth aspect the present invention resides in a dispenser for dispensing an ozone containing foam, which optionally incorporates one or more features of one or more of the first to eighty seventh aspects, wherein the ozone adjustor adjusts at least one of: a concentration of ozone in the ozone containing gas generated by the ozone generator; a volume of the ozone containing gas that is mixed with the foamable liquid by the foam generator; a ratio of the foamable liquid to the ozone containing gas; and a volume of the ozone containing foam that is dispensed from the discharge outlet.

In an eighty ninth aspect the present invention resides in a dispenser for dispensing an ozone containing foam, which optionally incorporates one or more features of one or more of the first to eighty eighth aspects, wherein the ozone adjustor allows for selection of the amount of ozone that is dispensed in the ozone containing foam at least from: a first amount of ozone that is selected to treat or prevent dermatitis; and a second amount of ozone that is selected to kill pathogens.

In a ninetieth aspect the present invention resides in a dispenser for dispensing an ozone containing foam, which optionally incorporates one or more features of one or more of the first to eighty ninth aspects, wherein the ozone adjustor allows for selection of an ozone-free mode of operation, wherein an ozone-free fluid is dispensed from the dispenser.

In a ninety first aspect the present invention resides in a dispenser for dispensing an ozone containing foam, which optionally incorporates one or more features of one or more of the first to ninetieth aspects, wherein, when in the ozone-free mode of operation, the foam generator receives an ozone-free gas and mixes the ozone-free gas with the foamable liquid to generate an ozone-free foam; and wherein the ozone-free foam is discharged from the discharge outlet.

In a ninety second aspect the present invention resides in a dispenser for dispensing an ozone containing foam, which optionally incorporates one or more features of one or more of the first to ninety first aspects, further comprising: a reservoir containing a supply of the foamable liquid; a liquid pump for delivering the foamable liquid to the foam generator; and a gas pump for delivering the ozone containing gas to the foam generator.

In a ninety third aspect the present invention resides in a dispenser for dispensing an ozone containing foam, which optionally incorporates one or more features of one or more of the first to ninety second aspects, further comprising a sensor that senses a concentration of ozone in the ozone containing gas, or that senses a parameter for estimating or calculating the concentration of ozone in the ozone containing gas.

In a ninety fourth aspect the present invention resides in a dispenser for dispensing an ozone containing foam, which optionally incorporates one or more features of one or more of the first to ninety third aspects, further comprising a controller that controls the ozone generator so that the concentration of ozone in the ozone containing gas is within a preselected range of concentrations.

In a ninety fifth aspect the present invention resides in a dispenser for dispensing an ozone containing foam, which optionally incorporates one or more features of one or more of the first to ninety fourth aspects, wherein the foamable liquid is a hand disinfectant.

In a ninety sixth aspect the present invention resides in a dispenser for dispensing an ozone containing foam, which optionally incorporates one or more features of one or more of the first to ninety fifth aspects, wherein the foam contains alcohol for disinfecting a user's hand.

In a ninety seventh aspect the present invention resides in a dispenser for dispensing an ozone containing foam, which optionally incorporates one or more features of one or more of the first to ninety sixth aspects, wherein the foam comprises bubbles of the gas in the foamable liquid.

In a ninety eighth aspect the present invention resides in a dispenser for dispensing an ozone containing foam, which optionally incorporates one or more features of one or more of the first to ninety seventh aspects, wherein the foam is selected to remain stable for a preselected period of time.

In a ninety ninth aspect the present invention resides in a dispenser for dispensing an ozone containing foam, which optionally incorporates one or more features of one or more of the first to ninety eighth aspects, wherein at least one of the gas, the foamable liquid, a size of the bubbles, a ratio of the foamable liquid to the gas, and a method of generating the foam are selected so that at least some of the bubbles remain intact for the preselected period of time.

In a one hundredth aspect the present invention resides in a dispenser for dispensing an ozone containing foam, which optionally incorporates one or more features of one or more of the first to ninety ninth aspects, wherein at least 50% of the bubbles of gas remain intact for at least 30 seconds.

In a one hundred and first aspect the present invention resides in a dispenser for dispensing an ozone containing foam, which optionally incorporates one or more features of one or more of the first to one hundredth aspects, wherein the bubbles are smaller than 2 mm.

In a one hundred and second aspect the present invention resides in a dispenser for dispensing an ozone containing foam, which optionally incorporates one or more features of one or more of the first to one hundred and first aspects, wherein the bubbles are smaller than 1 mm.

In a one hundred and third aspect the present invention resides in a dispenser for dispensing an ozone containing foam, which optionally incorporates one or more features of one or more of the first to one hundred and second aspects, wherein the bubbles have a mean bubble diameter in a range of 50 µm to 600 µm.

In a one hundred and fourth aspect the present invention resides in a dispenser for dispensing an ozone containing foam, which optionally incorporates one or more features of one or more of the first to one hundred and third aspects, wherein the bubbles have a mean bubble diameter in a range of 100 µm to 300 µm.

In a one hundred and fifth aspect the present invention resides in a dispenser for dispensing an ozone containing foam, which optionally incorporates one or more features of one or more of the first to one hundred and fourth aspects, wherein the foam has a ratio of about 1:5 of the foamable liquid to the gas by volume.

In a one hundred and sixth aspect the present invention resides in a dispenser for dispensing an ozone containing foam, which optionally incorporates one or more features of one or more of the first to one hundred and fifth aspects, wherein the foam has a ratio of about 1:10 of the foamable liquid to the gas by volume.

In a one hundred and seventh aspect the present invention resides in a dispenser for dispensing an ozone containing foam, which optionally incorporates one or more features of one or more of the first to one hundred and sixth aspects, wherein the foam has a ratio of about 1:15 of the foamable liquid to the gas by volume.

In a one hundred and eighth aspect the present invention resides in a dispenser for dispensing an ozone containing foam, which optionally incorporates one or more features of one or more of the first to one hundred and seventh aspects, wherein the foam has a ratio of about 1:50 of the foamable liquid to the gas by volume.

In a one hundred and ninth aspect the present invention resides in a dispenser for dispensing an ozone containing foam, which optionally incorporates one or more features of one or more of the first to one hundred and eighth aspects, wherein the foam has a ratio of between about 1:5 and about 1:60 of the foamable liquid to the gas by volume.

In a one hundred and tenth aspect the present invention resides in a dispenser for dispensing an ozone containing foam, which optionally incorporates one or more features of one or more of the first to one hundred and ninth aspects, wherein the foam comprises about 0.5 mL of the foamable liquid and about 2.5 mL of the gas.

In a one hundred and eleventh aspect the present invention resides in a dispenser for dispensing an ozone containing foam, which optionally incorporates one or more features of one or more of the first to one hundred and tenth aspects, wherein the foam comprises about 1 mL of the foamable liquid and about 5 mL of the gas.

In a one hundred and twelfth aspect the present invention resides in a dispenser for dispensing an ozone containing foam, which optionally incorporates one or more features of one or more of the first to one hundred and eleventh aspects, wherein the foam comprises about 1 mL of the foamable liquid and about 10 mL of the gas.

In a one hundred and thirteenth aspect the present invention resides in a dispenser for dispensing an ozone containing foam, which optionally incorporates one or more features of one or more of the first to one hundred and twelfth aspects, wherein the foam comprises between about 0.5 mL and 10 mL of the foamable liquid and between about 2.5 mL and 100 mL of the gas.

In a one hundred and fourteenth aspect the present invention resides in a dispenser for dispensing an ozone containing foam, which optionally incorporates one or more features of one or more of the first to one hundred and thirteenth aspects, wherein the foamable liquid comprises at least one of: ethanol, isopropanol, n-propanol, and mixtures thereof.

In a one hundred and fifteenth aspect the present invention resides in a dispenser for dispensing an ozone containing foam, which optionally incorporates one or more features of one or more of the first to one hundred and fourteenth aspects, wherein the foamable liquid comprises about 20 to 90% alcohol by volume and about 80 to 10% water by volume.

In a one hundred and sixteenth aspect the present invention resides in a dispenser for dispensing an ozone containing foam, which optionally incorporates one or more features of one or more of the first to one hundred and fifteenth aspects, wherein the foamable liquid comprises a foaming agent.

In a one hundred and seventeenth aspect the present invention resides in a dispenser for dispensing an ozone containing foam, which optionally incorporates one or more features of one or more of the first to one hundred and sixteenth aspects, wherein the foaming agent comprises a surfactant.

In a one hundred and eighteenth aspect the present invention resides in a dispenser for dispensing an ozone containing foam, which optionally incorporates one or more features of one or more of the first to one hundred and seventeenth aspects, wherein the foamable liquid comprises about 0.1% to 15% of the surfactant by volume.

In a one hundred and nineteenth aspect the present invention resides in a dispenser for dispensing an ozone containing foam, which optionally incorporates one or more features of one or more of the first to one hundred and eighteenth aspects, wherein the foam is for being applied to a user's hand.

In a one hundred and twentieth aspect the present invention resides in a dispenser for dispensing an ozone containing foam, which optionally incorporates one or more features of one or more of the first to one hundred and nineteenth aspects, wherein the foam is for being applied into an inner cavity of a glove.

In a one hundred and twenty first aspect the present invention resides in a dispenser for dispensing an ozone containing foam, which optionally incorporates one or more features of one or more of the first to one hundred and twentieth aspects, wherein the foam contains 2 to 300 µM of ozone.

In a one hundred and twenty second aspect the present invention resides in a dispenser for dispensing an ozone containing foam, which optionally incorporates one or more features of one or more of the first to one hundred and twenty first aspects, wherein the foam contains 10 to 40 µg/mL of ozone.

In a one hundred and twenty third aspect the present invention resides in a dispenser for dispensing an ozone containing foam, which optionally incorporates one or more features of one or more of the first to one hundred and twenty second aspects, wherein the ozone containing gas has less than 0.05% ozone by volume.

In a one hundred and twenty fourth aspect the present invention resides in a dispenser for dispensing an ozone containing foam, which optionally incorporates one or more features of one or more of the first to one hundred and twenty third aspects, wherein the ozone containing gas has less than 0.04% ozone by volume.

In a one hundred and twenty fifth aspect the present invention resides in a dispenser for dispensing an ozone containing foam, which optionally incorporates one or more features of one or more of the first to one hundred and twenty fourth aspects, wherein the ozone containing gas has less than 0.01% ozone by volume.

In a one hundred and twenty sixth aspect the present invention resides in a dispenser for dispensing an ozone containing foam, which optionally incorporates one or more features of one or more of the first to one hundred and twenty fifth aspects, wherein the ozone containing gas has less than 0.005% ozone by volume.

In a one hundred and twenty seventh aspect the present invention resides in a dispenser for dispensing an ozone containing foam, which optionally incorporates one or more features of one or more of the first to one hundred and twenty sixth aspects, wherein the ozone containing gas has less than 0.001% ozone by volume.

In a one hundred and twenty eighth aspect the present invention resides in a dispenser for dispensing an ozone containing foam, which optionally incorporates one or more features of one or more of the first to one hundred and twenty seventh aspects, wherein the ozone containing gas has less than 0.0001% ozone by volume.

In a one hundred and twenty ninth aspect the present invention resides in a dispenser for dispensing an ozone containing foam, which optionally incorporates one or more features of one or more of the first to one hundred and twenty eighth aspects, wherein the ozone containing gas has less than 0.00001% ozone by volume.

In a one hundred and thirtieth aspect the present invention resides in a dispenser for dispensing an ozone containing foam, which optionally incorporates one or more features of one or more of the first to one hundred and twenty ninth aspects, wherein the ozone containing gas has less than 0.000001% ozone by volume.

In a one hundred and thirty first aspect the present invention resides in a dispenser for dispensing an ozone containing foam, which optionally incorporates one or more features of one or more of the first to one hundred and thirtieth aspects, wherein the ozone containing gas has between 0.000001% and 0.05% ozone by volume.

In a one hundred and thirty second aspect the present invention resides in a dispenser for dispensing an ozone containing foam, which optionally incorporates one or more features of one or more of the first to one hundred and thirty first aspects, wherein the ozone containing gas comprises ozonated air.

In a one hundred and thirty third aspect the present invention resides in a hand hygiene station, which optionally incorporates one or more features of one or more of the first to one hundred and thirty second aspects, comprising: a foam dispenser for dispensing foam; and a glove dispenser for dispensing gloves; wherein the foam is for at least one of: assisting in sliding a user's hand into one of the gloves; treating dermatitis; and preventing dermatitis.

In a one hundred and thirty fourth aspect the present invention resides in a hand hygiene station, which optionally incorporates one or more features of one or more of the first to one hundred and thirty third aspects, wherein the foam is for assisting in sliding the user's hand into one of the gloves.

In a one hundred and thirty fifth aspect the present invention resides in a hand hygiene station, which optionally incorporates one or more features of one or more of the first to one hundred and thirty fourth aspects, wherein the foam is for treating dermatitis.

In a one hundred and thirty sixth aspect the present invention resides in a hand hygiene station, which optionally incorporates one or more features of one or more of the first to one hundred and thirty fifth aspects, wherein the foam is for preventing dermatitis.

In a one hundred and thirty seventh aspect the present invention resides in a hand hygiene station, which optionally incorporates one or more features of one or more of the first to one hundred and thirty sixth aspects, wherein the foam is also for disinfecting the user's hand.

In a one hundred and thirty eighth aspect the present invention resides in a hand hygiene station, which optionally incorporates one or more features of one or more of the first to one hundred and thirty seventh aspects, wherein the foam contains ozone.

In a one hundred and thirty ninth aspect the present invention resides in a hand hygiene station, which optionally incorporates one or more features of one or more of the first to one hundred and thirty eighth aspects, wherein the foam dispenser is for delivering the foam into the gloves.

In a one hundred and fortieth aspect the present invention resides in a hand hygiene station, which optionally incorporates one or more features of one or more of the first to one hundred and thirty ninth aspects, wherein the foam dispenser comprises the dispenser in accordance with in any one of the eighty sixth to one hundred and thirty third aspects.

In a one hundred and forty first aspect the present invention resides in a method of placing a glove onto a hand, which optionally incorporates one or more features of one or more of the first to one hundred and fortieth aspects, comprising: coating the hand in foam; and sliding the hand, while the hand is coated in the foam, into the glove.

In a one hundred and forty second aspect the present invention resides in a method, which optionally incorporates one or more features of one or more of the first to one hundred and forty first aspects, wherein the foam contains ozone.

In a one hundred and forty third aspect the present invention resides in a method, which optionally incorporates one or more features of one or more of the first to one hundred and forty second aspects, wherein the foam has a concentration of ozone that is selected to kill pathogens on the hand.

In a one hundred and forty fourth aspect the present invention resides in a method, which optionally incorporates one or more features of one or more of the first to one hundred and forty third aspects, wherein the foam disinfects the hand.

In a one hundred and forty fifth aspect the present invention resides in a method, which optionally incorporates one or more features of one or more of the first to one hundred and forty fourth aspects, wherein the foam contains alcohol for disinfecting the hand.

In a one hundred and forty sixth aspect the present invention resides in a method, which optionally incorporates one or more features of one or more of the first to one hundred and forty fifth aspects, wherein the foam is selected to remain stable for a preselected period of time on the hand.

In a one hundred and forty seventh aspect the present invention resides in a method, which optionally incorporates one or more features of one or more of the first to one hundred and forty sixth aspects, wherein the foam comprises bubbles of gas in a foamable liquid; and wherein at least one of the gas, the foamable liquid, a size of the bubbles, a ratio of the foamable liquid to the gas, and a method of generating the foam are selected so that at least some of the bubbles remain intact for the preselected period of time on the hand.

In a one hundred and forty eighth aspect the present invention resides in a method, which optionally incorporates one or more features of one or more of the first to one hundred and forty seventh aspects, wherein at least 50% of the bubbles of gas remain intact for at least 30 seconds on the hand.

In a one hundred and forty ninth aspect the present invention resides in a method of placing a glove onto a hand, which optionally incorporates one or more features of one or more of the first to one hundred and forty eighth aspects, comprising: applying foam into an inner cavity of the glove; and sliding the hand into the glove.

In a one hundred and fiftieth aspect the present invention resides in a method, which optionally incorporates one or more features of one or more of the first to one hundred and forty ninth aspects, wherein the foam contains ozone.

In a one hundred and fifty first aspect the present invention resides in a method, which optionally incorporates one or more features of one or more of the first to one hundred and fiftieth aspects, wherein the foam has a concentration of ozone that is selected to kill pathogens on the hand.

In a one hundred and fifty second aspect the present invention resides in a method, which optionally incorporates one or more features of one or more of the first to one hundred and fifty first aspects, wherein the foam disinfects the hand.

In a one hundred and fifty third aspect the present invention resides in a method, which optionally incorporates one or more features of one or more of the first to one hundred and fifty second aspects, wherein the foam contains alcohol for disinfecting the hand.

In a one hundred and fifty fourth aspect the present invention resides in a method, which optionally incorporates one or more features of one or more of the first to one hundred and fifty third aspects, wherein the foam is selected to remain stable for a preselected period of time.

In a one hundred and fifty fifth aspect the present invention resides in a method, which optionally incorporates one or more features of one or more of the first to one hundred and fifty fourth aspects, wherein the foam comprises bubbles of gas in a foamable liquid; and wherein at least one of the gas, the foamable liquid, a size of the bubbles, a ratio of the foamable liquid to the gas, and a method of generating the foam are selected so that at least some of the bubbles remain intact for the preselected period of time.

In a one hundred and fifty sixth aspect the present invention resides in a method, which optionally incorporates one or more features of one or more of the first to one hundred and fifty fifth aspects, wherein at least 50% of the bubbles of gas remain intact for at least 30 seconds.

In a one hundred and fifty seventh aspect the present invention resides in use of an ozone containing foam, which optionally incorporates one or more features of one or more of the first to one hundred and fifty sixth aspects, wherein the ozone containing foam is used to treat dermatitis on a hand.

In a one hundred and fifty eighth aspect the present invention resides in use of an ozone containing foam, which optionally incorporates one or more features of one or more of the first to one hundred and fifty seventh aspects, wherein the ozone containing foam is used to prevent dermatitis on a hand.

In a one hundred and fifty ninth aspect the present invention resides in a method of placing a glove onto a hand, which optionally incorporates one or more features of one or more of the first to one hundred and fifty eighth aspects, comprising: at least one of: coating the hand in foam; and applying foam into an inner cavity of the glove; and sliding the hand into the glove.

In a one hundred and sixtieth aspect the present invention resides in a method, which optionally incorporates one or more features of one or more of the first to one hundred and fifty ninth aspects, wherein the foam is applied into the inner cavity of the glove before sliding the hand into the glove.

In a one hundred and sixty first aspect the present invention resides in a method, which optionally incorporates one or more features of one or more of the first to one hundred and sixtieth aspects, wherein the foam contains ozone.

In a one hundred and sixty second aspect the present invention resides in a method, which optionally incorporates one or more features of one or more of the first to one hundred and sixty first aspects, wherein the foam has a concentration of ozone that is selected to treat dermatitis on the hand.

In a one hundred and sixty third aspect the present invention resides in a method, which optionally incorporates one or more features of one or more of the first to one hundred and sixty second aspects, wherein the foam has a concentration of ozone that is selected to protect the hand from dermatitis.

In a one hundred and sixty fourth aspect the present invention resides in a method, which optionally incorporates one or more features of one or more of the first to one hundred and sixty third aspects, wherein the foam has a concentration of ozone that is selected to promote healing of the hand.

In a one hundred and sixty fifth aspect the present invention resides in a method, which optionally incorporates one or more features of one or more of the first to one hundred and sixty fourth aspects, wherein the foam has a concentration of ozone that is selected to kill pathogens on the hand.

In a one hundred and sixty sixth aspect the present invention resides in a method, which optionally incorporates one or more features of one or more of the first to one hundred and sixty fifth aspects, wherein the foam disinfects the hand.

In a one hundred and sixty seventh aspect the present invention resides in a method, which optionally incorporates one or more features of one or more of the first to one hundred and sixty sixth aspects, wherein the foam contains alcohol for disinfecting the hand.

In a one hundred and sixty eighth aspect the present invention resides in a method, which optionally incorporates one or more features of one or more of the first to one hundred and sixty seventh aspects, wherein the foam is selected to remain stable for a preselected period of time.

In a one hundred and sixty ninth aspect the present invention resides in a method, which optionally incorporates one or more features of one or more of the first to one hundred and sixty eighth aspects, wherein the foam comprises bubbles of gas in a foamable liquid; and wherein at least one of the gas, the foamable liquid, a size of the bubbles, a ratio of the foamable liquid to the gas, and a method of generating the foam are selected so that at least some of the bubbles remain intact for the preselected period of time.

In a one hundred and seventieth aspect the present invention resides in a method, which optionally incorporates one or more features of one or more of the first to one hundred and sixty ninth aspects, wherein at least 50% of the bubbles of gas remain intact for at least 30 seconds.

In a one hundred and seventy first aspect the present invention resides in a method, which optionally incorporates one or more features of one or more of the first to one hundred and seventieth aspects, wherein the foam comprises bubbles of an ozonated gas in a foamable liquid.

In a one hundred and seventy second aspect the present invention resides in a method, which optionally incorporates one or more features of one or more of the first to one hundred and seventy first aspects, wherein the bubbles have a mean bubble diameter in a range of 50 µm to 600 µm.

In a one hundred and seventy third aspect the present invention resides in a method, which optionally incorporates one or more features of one or more of the first to one hundred and seventy second aspects, wherein the foam has a ratio of between about 1:5 and about 1:60 of the foamable liquid to the ozonated gas by volume.

In a one hundred and seventy fourth aspect the present invention resides in a method, which optionally incorporates one or more features of one or more of the first to one hundred and seventy third aspects, wherein the foam comprises between about 0.5 mL and 10 mL of the foamable liquid and between about 2.5 mL and 100 mL of the ozonated gas.

In a one hundred and seventy fifth aspect the present invention resides in a method, which optionally incorporates one or more features of one or more of the first to one hundred and seventy fourth aspects, wherein the foamable liquid comprises a foaming agent; wherein the foaming agent comprises a surfactant; and wherein the foamable liquid comprises about 0.1% to 15% of the surfactant by volume.

In a one hundred and seventy sixth aspect the present invention resides in a method, which optionally incorporates one or more features of one or more of the first to one hundred and seventy fifth aspects, wherein the ozonated gas has between 0.000001% and 0.05% ozone by volume.

In a one hundred and seventy seventh aspect the present invention resides in a method, which optionally incorporates one or more features of one or more of the first to one hundred and seventy sixth aspects, wherein the foam has a concentration of ozone that is selected to treat dermatitis on the hand and/or to protect the hand from dermatitis; wherein the foam contains alcohol for disinfecting the hand; and wherein the ozonated gas comprises ozonated air.

In a one hundred and seventy eighth aspect the present invention resides in a method, which optionally incorporates one or more features of one or more of the first to one hundred and seventy seventh aspects, wherein the foam is selected to remain stable for a preselected period of time; wherein at least 50% of the bubbles of gas remain intact for at least 30 seconds; wherein the bubbles have a mean bubble diameter in a range of 50 µm to 600 µm; wherein the foam has a ratio of between about 1:5 and about 1:60 of the foamable liquid to the ozonated gas by volume; wherein the foam comprises between about 0.5 mL and 10 mL of the foamable liquid and between about 2.5 mL and 100 mL of the ozonated gas; wherein the ozonated gas has between 0.000001% and 0.05% ozone by volume; wherein the foamable liquid comprises a foaming agent; wherein the foaming agent comprises a surfactant; and wherein the foamable liquid comprises about 0.1% to 15% of the surfactant by volume.

In a one hundred and seventy ninth aspect the present invention resides in a method of treating dermatitis, which optionally incorporates one or more features of one or more of the first to one hundred and seventy eighth aspects, comprising: applying an ozone containing foam to a person's skin.

In a one hundred and eightieth aspect the present invention resides in a method, which optionally incorporates one or more features of one or more of the first to one hundred and seventy ninth aspects, wherein the ozone containing foam is applied to a hand.

In a one hundred and eighth first aspect the present invention resides in a method of preventing dermatitis, which optionally incorporates one or more features of one or more of the first to one hundred and eightieth aspects, comprising: applying an ozone containing foam to a person's skin.

In a one hundred and eighty second aspect the present invention resides in a method, which optionally incorporates one or more features of one or more of the first to one hundred and eighty first aspects, wherein the ozone containing foam is applied to a hand.

In a one hundred and eighty third aspect the present invention resides in a method of treating dermatitis on a hand, which optionally incorporates one or more features of one or more of the first to one hundred and eighty second aspects, comprising: placing an ozone containing foam into a glove; and placing the hand into the glove.

In a one hundred and eighty fourth aspect the present invention resides in a method of preventing dermatitis on a hand, which optionally incorporates one or more features of one or more of the first to one hundred and eighty third aspects, comprising: placing an ozone containing foam into a glove; and placing the hand into the glove.

In a one hundred and eighty fifth aspect the present invention resides in a dispenser for dispensing an ozone containing foam, which optionally incorporates one or more features of one or more of the first to one hundred and eighty fourth aspects, the dispenser comprising: an ozone generator that generates an ozone containing gas; a foam generator that receives the ozone containing gas and mixes the ozone containing gas with a foamable liquid to generate the ozone containing foam; and a discharge outlet for discharging the ozone containing foam.

In a one hundred and eighty sixth aspect the present invention resides in a foam for use in assisting in sliding a hand into a glove, which optionally incorporates one or more features of one or more of the first to one hundred and eighty fifth aspects.

In a one hundred and eighty seventh aspect the present invention resides in a foam, which optionally incorporates one or more features of one or more of the first to one hundred and eighty sixth aspects, wherein the foam contains ozone.

In a one hundred and eighty eighth aspect the present invention resides in a foam, which optionally incorporates one or more features of one or more of the first to one hundred and eighty seventh aspects, wherein the foam has a concentration of ozone that is selected to treat dermatitis on the hand.

In a one hundred and eighty ninth aspect the present invention resides in a foam, which optionally incorporates one or more features of one or more of the first to one hundred and eighty eighth aspects, wherein the foam has a concentration of ozone that is selected to protect the hand from dermatitis.

In a one hundred and ninetieth aspect the present invention resides in a foam, which optionally incorporates one or more features of one or more of the first to one hundred and eighty ninth aspects, wherein the foam has a concentration of ozone that is selected to promote healing of the hand.

In a one hundred and ninety first aspect the present invention resides in a foam, which optionally incorporates one or more features of one or more of the first to one hundred and ninetieth aspects, wherein the foam has a concentration of ozone that is selected to kill pathogens on the hand.

In a one hundred and ninety second aspect the present invention resides in a foam, which optionally incorporates one or more features of one or more of the first to one hundred and ninety first aspects, wherein the foam contains alcohol for disinfecting the hand.

In a one hundred and ninety third aspect the present invention resides in a foam, which optionally incorporates one or more features of one or more of the first to one hundred and ninety second aspects, wherein the foam comprises bubbles of an ozonated gas in a foamable liquid.

In a one hundred and ninety fourth aspect the present invention resides in a foam, which optionally incorporates one or more features of one or more of the first to one hundred and ninety third aspects, wherein at least 50% of the bubbles remain intact for at least 30 seconds on the hand.

In a one hundred and ninety fifth aspect the present invention resides in a foam, which optionally incorporates one or more features of one or more of the first to one hundred and ninety fourth aspects, wherein the bubbles have a mean bubble diameter in a range of 50 µm to 600 µm.

In a one hundred and ninety sixth aspect the present invention resides in a foam, which optionally incorporates one or more features of one or more of the first to one hundred and ninety fifth aspects, wherein the foam has a ratio of between about 1:5 and about 1:60 of the foamable liquid to the ozonated gas by volume.

In a one hundred and ninety seventh aspect the present invention resides in a foam, which optionally incorporates one or more features of one or more of the first to one hundred and ninety sixth aspects, wherein the foam comprises between about 0.5 mL and 10 mL of the foamable liquid and between about 2.5 mL and 100 mL of the ozonated gas.

In a one hundred and ninety eighth aspect the present invention resides in a foam, which optionally incorporates one or more features of one or more of the first to one hundred and ninety seventh aspects, wherein the foamable liquid comprises a foaming agent; wherein the foaming agent comprises a surfactant; and wherein the foamable liquid comprises about 0.1% to 15% of the surfactant by volume.

In a one hundred and ninety ninth aspect the present invention resides in a foam, which optionally incorporates one or more features of one or more of the first to one hundred and ninety eighth aspects, wherein the ozonated gas has between 0.000001% and 0.05% ozone by volume.

In a two hundredth aspect the present invention resides in a foam, which optionally incorporates one or more features of one or more of the first to one hundred and ninety ninth aspects, wherein the foamable liquid is a hand disinfectant; and wherein the ozonated gas comprises ozonated air.

In a two hundred and first aspect the present invention resides in an ozone containing foam for use in treating dermatitis, which optionally incorporates one or more features of one or more of the first to two hundredth aspects.

In a two hundred and second aspect the present invention resides in an ozone containing foam, which optionally incorporates one or more features of one or more of the first to two hundred and first aspects, wherein the ozone containing foam is for treating dermatitis on a hand.

In a two hundred and third aspect the present invention resides in an ozone containing foam for use in preventing dermatitis, which optionally incorporates one or more features of one or more of the first to two hundred and second aspects.

In a two hundred and fourth aspect the present invention resides in an ozone containing foam, which optionally incorporates one or more features of one or more of the first to two hundred and third aspects, wherein the ozone containing foam is for preventing dermatitis on a hand.

In a two hundred and fifth aspect the present invention resides in an ozone containing foam, which optionally incorporates one or more features of one or more of the first to two hundred and fourth aspects, wherein the ozone containing foam is inside a glove.

In a two hundred and sixth aspect the present invention resides in an ozone containing foam, which optionally incorporates one or more features of one or more of the first to two hundred and fifth aspects, wherein the ozone containing foam contains alcohol for disinfecting the hand.

In a two hundred and seventh aspect the present invention resides in an ozone containing foam, which optionally incorporates one or more features of one or more of the first to two hundred and sixth aspects, wherein the ozone containing foam comprises bubbles of an ozonated gas in a foamable liquid.

In a two hundred and eighth aspect the present invention resides in an ozone containing foam, which optionally incorporates one or more features of one or more of the first to two hundred and seventh aspects, wherein at least 50% of the bubbles remain intact for at least 30 seconds on the hand.

In a two hundred and ninth aspect the present invention resides in an ozone containing foam, which optionally incorporates one or more features of one or more of the first to two hundred and eighth aspects, wherein the bubbles have a mean bubble diameter in a range of 50 µm to 600 µm.

In a two hundred and tenth aspect the present invention resides in an ozone containing foam, which optionally incorporates one or more features of one or more of the first to two hundred and ninth aspects, wherein the foam has a ratio of between about 1:5 and about 1:60 of the foamable liquid to the ozonated gas by volume.

In a two hundred and eleventh aspect the present invention resides in an ozone containing foam, which optionally incorporates one or more features of one or more of the first to two hundred and tenth aspects, wherein the foam comprises between about 0.5 mL and 10 mL of the foamable liquid and between about 2.5 mL and 100 mL of the ozonated gas.

In a two hundred and twelfth aspect the present invention resides in an ozone containing foam, which optionally incorporates one or more features of one or more of the first to two hundred and eleventh aspects, wherein the foamable liquid comprises a foaming agent; wherein the foaming agent comprises a surfactant; and wherein the foamable liquid comprises about 0.1% to 15% of the surfactant by volume.

In a two hundred and thirteenth aspect the present invention resides in an ozone containing foam, which optionally incorporates one or more features of one or more of the first to two hundred and twelfth aspects, wherein the ozonated gas has between 0.000001% and 0.05% ozone by volume.

In a two hundred and fourteenth aspect the present invention resides in an ozone containing foam, which optionally incorporates one or more features of one or more of the first to two hundred and thirteenth aspects, wherein the foamable liquid is a hand disinfectant; and wherein the ozonated gas comprises ozonated air.

### Brief Description of the Drawings

Further aspects and advantages of the invention will appear from the following description taken together with the accompanying drawings, in which:
Figure 1 is a schematic representation of a foam dispenser in accordance with a first embodiment of the present invention;
Figure 2 is a side view of the foam dispenser shown in Figure 1, with the internal components of the foam dispenser hidden from view within a housing;
Figure 3 is a side view of a user's hand that is coated with foam to assist in placing a glove onto the user's hand;
Figure 4 is a side view of the foam dispenser shown in Figure 2 dispensing foam into a glove; and
Figure 5 is a perspective front view of a hand hygiene station in accordance with a second embodiment of the present invention.

### Detailed Description of the Drawings

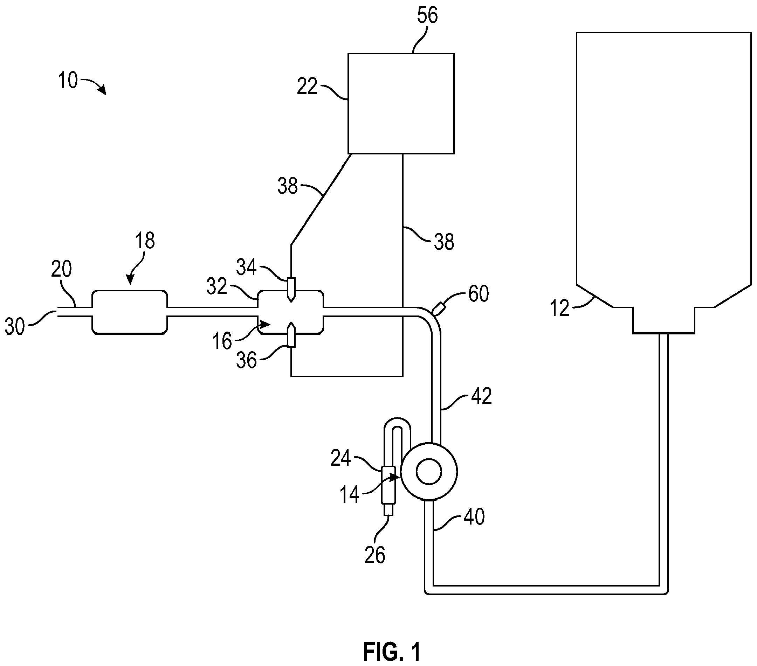

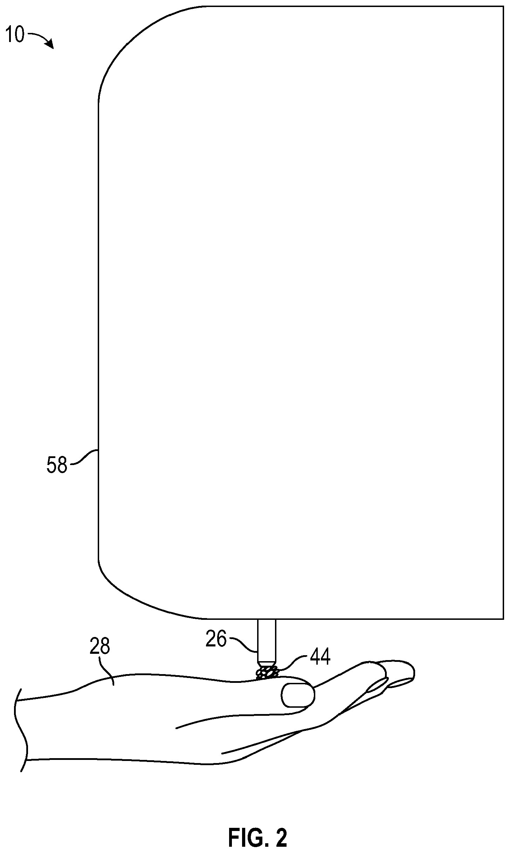

Figures 1 and 2 show a foam dispenser 10 in accordance with a first embodiment of the present invention. The foam dispenser 10 is operative to discharge an ozone containing foam 44 from a foam outlet 26 onto a user's hand 28. The internal components of the foam dispenser 10 are shown schematically in Figure 1, and are hidden from view within a housing 58 in Figure 2. As shown in Figure 1, the foam dispenser 10 has a fluid reservoir 12, a pump 14, an ozone generator 16, a desiccant 18, an air inlet tube 20, a controller 22, a foam generator 24, and a foam outlet 26. The fluid reservoir 12 contains a foamable liquid. The foamable liquid is preferably a hand sanitizing liquid that may, for example, contain alcohol for the purpose of disinfecting/sanitizing a user's hand 28. The fluid reservoir 12 is in fluid communication with a liquid inlet 40 of the pump 14 for delivering the foamable liquid to the pump 14.

The air inlet tube 20 has an open end 30 that is open to the atmospheric air. The desiccant 18 is in fluid communication with the air inlet tube 20, and serves to remove moisture from atmospheric air that is drawn through the desiccant 18 from the open end 30. The desiccant 18 is in fluid communication with the ozone generator 16 for delivering the dried atmospheric air to the ozone generator 16.

The ozone generator 16 is preferably in the form of a corona discharge chamber 32 having a first electrode 34 and a second electrode 36. The first electrode 34 and the second electrode 36 are connected by wires 38 to the controller 22. The controller 22 is configured to activate the ozone generator 16 by applying a sufficiently high voltage between the first electrode 34 and the second electrode 36 to generate a corona discharge. When the ozone generator 16 is activated, the corona discharge converts oxygen in the dried atmospheric air received from the desiccant 18 into ozone, thereby producing ozonated air. The ozone generator 16 is in fluid communication with an air inlet 42 of the pump 14 for delivering the ozonated air to the pump 14.

The pump 14 is configured to draw the foamable liquid from the fluid reservoir 12 into the pump 14 through the liquid inlet 40; to draw the ozonated air from the ozone generator 16 into the pump 14 through the air inlet 42; and is in fluid communication with the foam generator 24 for delivering the foamable liquid and the ozonated air from the pump 14 to the foam generator 24. The foam generator 24 is configured to thoroughly mix the foamable liquid and the ozonated air to generate an ozone containing foam 44, which is then discharged from the foam outlet 26. The ozone containing foam 44 is preferably discharged from the foam outlet 26 onto a user's hand 28, as shown in Figure 2.

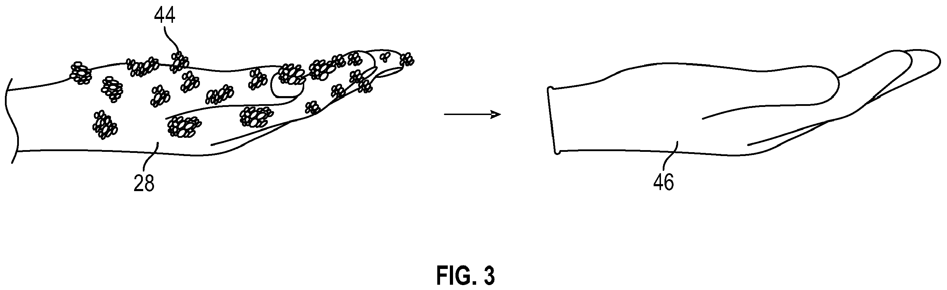

After the foam 44 is dispensed onto the user's hand 28, it is preferably spread over the entire outer surface of the user's hand 28 by, for example, rubbing the user's hands 28 together, so that there is a coating of foam 44 covering the user's hand 28 as shown in Figure 3. With the foam 44 covering the user's hand 28, the alcohol in the foam 44 disinfects the user's hand 28 by killing some or all of the pathogens that are on the user's hand 28. The ozone in the foam 44 also preferably helps to treat and/or protect against dermatitis on the user's hand 28. The ozone may, for example, react with molecules on the surface of the user's hand 28, providing a transient and mild oxidative stress that activates the body's natural defense mechanisms and repair mechanisms.

Preferably, while the hand 28 is coated with the foam 44, the hand 28 is placed inside a glove 46, as shown in Figure 3. The foam 44 may help to reduce the friction between the user's hand 28 and the glove 46, thus making it easier to put on the glove 46, and may help to reduce irritation of the user's skin.

### Ozone Concentration

The ozone containing foam 44 that is dispensed from the foam outlet 26 preferably contains a suitable quantity/concentration of ozone for treating dermatitis on the user's hand 28 and/or for protecting the user's hand 28 from dermatitis. The ozone containing foam 44 may, for example, contain a concentration from 2 to 300 µM of ozone, or a concentration from 10 to 40 µg/mL of ozone. Optionally, when the ozone containing foam 44 is to be used to treat dermatitis and/or to protect the user's hand 28 from dermatitis, the concentration of ozone in the ozonated air may be less than 0.05% (or 500 ppm) by volume; less than 0.04% (or 400 ppm) by volume; less than 0.03% (or 300 ppm) by volume; less than 0.02% (or 200 ppm) by volume; less than 0.01% (or 100 ppm) by volume; less than 0.005% (or 50 ppm) by volume; less than 0.001% (or 10 ppm) by volume; less than 0.0001% (or 1 ppm) by volume; less than 0.00001% (or 0.1 ppm) by volume; or between 0.000001% (or 0.01 ppm) and 0.05% (or 500 ppm) by volume. The concentration of ozone could also be greater than 0.05% (or 500 ppm) by volume. Higher concentrations of ozone may be desired, for example, if the ozone is to be used as a biocide or sanitizing agent. The dispenser 10 could also be configured to dispense foam 44 that contains no ozone, or which contains only a trace amount of ozone. Having little or no ozone may be desired, for example, if the foam 44 is not intended to treat dermatitis, and is provided only for the purpose of reducing friction between the user's hand 28 and the glove 46. The invention is not limited to any particular quantity/concentration of ozone, and a person skilled in the art could select any desired quantity/concentration of ozone that is suitable for the intended use of the foam 44.

### Foam Characteristics

The characteristics of the foam 44 may be selected based on its intended use, including foam height, foam bubble size, foam bubble size distribution, foam consistency, and foam stability or foam life time. Preferably, the foam 44 is formulated so as to remain relatively stable for at least a short duration of time after the foam 44 is dispensed, so that at least some of the bubbles in the foam 44 remain intact while the user spreads the foam 44 over his or her hands 28 and then puts on gloves 46. The foam 44 may, for example, be formulated so as to remain stable with at least some of the bubbles, and preferably most of the bubbles, intact for a preselected period of time. The foam 44 may, for example, remain stable with at least 25%, or at least 50%, or at least 75%, or at least 90%, or at least 95% of the bubbles intact for at least 5 seconds, or at least 10 seconds, or at least 20 seconds, or at least 30 seconds, or at least 1 minute, or at least 5 minutes after being dispensed.

Foam life time may be considered a measure of foam stability, and can be defined as a period in which the foam 44 remains after discharge as a foam 44 without collapsing into a liquid. Foam life time may be considered in one measure as foam half-life, being the period of time that one half of the gas bubbles remain without collapse into a liquid after discharge of the foam 44 onto a surface. The foam 44 may, for example, have a foam life time of at least 10 seconds, at least 30 seconds, at least 60 seconds, or greater. The foam half-life may, for example, be at least 20 seconds, at least 30 seconds, or greater.

In some embodiments of the invention, the foam 44 may be selected to have a relatively small bubble size, to improve the stability of the foam 44. The foam bubbles may, for example, be smaller than 2 mm or smaller than 1 mm. Optionally, the bubbles have a mean bubble diameter in a range of 50 µm to 600 µm, or in a range of 100 µm to 300 µm.

Other foam characteristics, such as the ratio of the foamable liquid to the ozonated air and the volume of the foam 44 that is dispensed with each activation of the dispenser 10, could also be selected based on the intended use of the foam 44. The ratio by volume of the foamable liquid to the ozonated air in the foam 44 when the foam 44 is dispensed could, for example, be about 1:5; about 1:10; about 1:15; about 1:50; about 1:60; or between about 1:5 and about 1:60. The volume of the foamable liquid and the ozonated air that are dispensed as a dose of foam 44 with each activation of the dispenser 10 could, for example, be about 0.5 mL of foamable liquid and about 2.5 mL of ozonated gas; or about 1 mL of foamable liquid and about 5 mL of ozonated gas; or about 1 mL of foamable liquid and about 10 mL of ozonated gas; or about 2 mL of foamable liquid and about 40 mL of ozonated gas; or about 2 mL of foamable liquid and about 80 mL of ozonated gas; or about 5 mL of foamable liquid and about 100 mL of ozonated gas; or about 10 mL of foamable liquid and about 100 mL of ozonated gas; or about 1 mL of foamable liquid and about 50 mL of ozonated gas; or about 5 mL of foamable liquid and about 25 mL of ozonated gas.

A person skilled in the art could adjust the formulation of the foam 44 so as to provide the desired degree of stability and other foam characteristics by, for example, adjusting the chemical composition or other properties of the ozone containing gas; the chemical composition or other properties of the foamable liquid; the size of the bubbles; the ratio of the gas to the foamable liquid; and the method by which the foam is generated, including for example the construction and material properties of the foam generator 24.

Foam characteristics that may be advantageous in some circumstances are described in US Patent Publication 2017/0231437 to Ciavarella, published August 17, 2017, and in US Patent 7,842,725 to Wegner et al., issued November 20, 2010, which are incorporated herein by reference.

### Foamable Liquid

Any suitable foamable liquid could be used, and the invention is not limited to any particular composition of the foamable liquid. The foamable liquid could, for example, contain water and an alcohol. The alcohol could, for example, be selected from ethanol, isopropanol, n-propanol, and mixtures thereof. The alcohol may, for example, comprise 20% to 90% by volume of the foamable liquid and the water may, for example, comprise 80% to 10% by volume of the foamable liquid. The foamable liquid also optionally includes an effective amount of a foaming agent, such as a surfactant. Suitable surfactants may include silicone containing compounds, surfactants containing silicon or silane, surfactants containing dimethicones, fluorosurfactants, and/or siloxane polymer surfactants. The surfactants may, for example, comprise 0.1% to 15% by volume of the foamable liquid. The foamable liquid may also contain other additives, such as foam stabilizers, emulsifying agents, conditioners, skin moisturizers, emollients, preservatives, fragrances, and dyes. Preferably, the foamable liquid does not include additives that would react with the ozone so as to reduce the concentration of ozone below the intended concentration. A person skilled in the art could adjust the composition of the foamable liquid based on its intended use.

### Other Optional Features and Uses

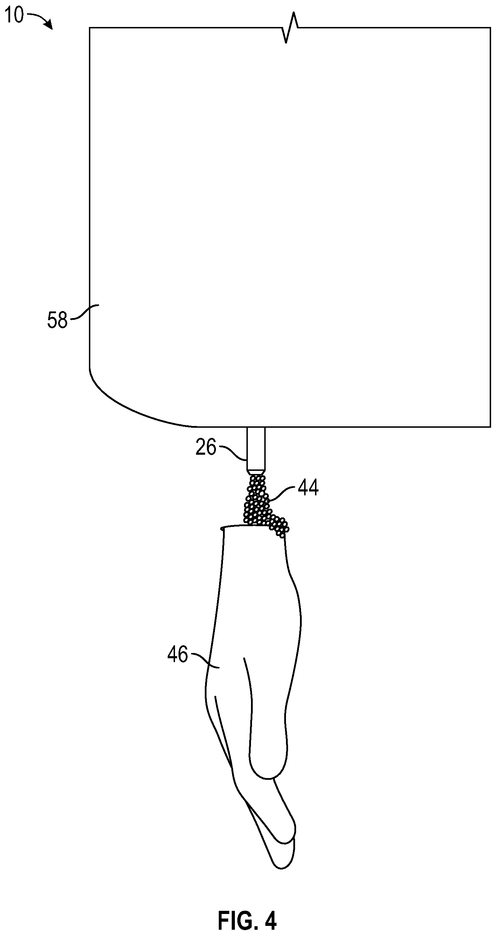

Optionally, the foam dispenser 10 could be used to dispense the ozone containing foam 44 directly into an inner cavity of the glove 44 before the glove 44 is put onto the user's hand 28, as shown in Figure 4. Dispensing the foam 44 into the glove 44 may help to reduce the friction between the user's hand 28 and the glove 44, thus making it easier to put on the glove 44 and potentially reducing skin irritation. Dispensing the foam 44 directly into the glove 44 could be done in addition to, or instead of, dispensing the foam 44 directly onto the user's hand 28.

Optionally, the controller 22 functions as an ozone adjuster 56 that is capable of adjusting the amount of ozone that is dispensed in the ozone containing foam 44 with each activation of the dispenser 10. The controller 22 may, for example, be configured to receive operating instructions from a user or input from a computer system to select a mode of operation from a set of possible modes of operation, with each mode of operation providing a different amount of ozone in the ozone containing foam 44. Any suitable mechanism for providing operating instructions to the controller 22 could be used, such as an electronic switch, a mechanical dial, a touchscreen interface, or a wireless connection. The controller 22 could, for example, allow for selection from a first mode of operation, in which the amount of ozone is selected to treat and/or prevent dermatitis; a second mode of operation, in which the amount of ozone is selected to kill pathogens on the user's hands 28; and a third mode of operation, in which the foam 44 is ozone-free. In the first mode of operation, the controller 22 could be configured to activate the ozone generator 16 for a relatively short amount of time, so that only a relatively low dose of ozone is generated, such as less than 0.05% of ozone by volume in the ozonated air. In the second mode of operation, the controller 22 could be configured to activate the ozone generator 16 for a longer period of time, so that a larger dose of ozone is generated, such as 0.1% of ozone by volume in the ozonated air. In the third mode of operation, the controller 22 could be configured to not activate the ozone generator 16 at all, so that no ozone is generated or dispensed.

The controller 22 could also be configured to adjust the amount of ozone that is dispensed from the dispenser 10 by, for example, adjusting the volume of the ozone containing gas that is mixed with the foamable liquid; the ratio of ozone containing gas to the foamable liquid; and/or the volume of the ozone containing foam 44 that is dispensed from the foam outlet 26. Any other suitable mechanism for adjusting the amount of ozone that is dispensed from the dispenser 10 in a single dosage could also be used.

Optionally, the dispenser 10 includes a sensor 60 that senses a concentration of ozone in the ozone containing gas that is generated by the ozone generator 16, or that senses a parameter that can be used to estimate or calculate the concentration of ozone in the ozone containing gas. The sensor 60 could be used, for example, to provide feedback to the controller 22 so that the operation of the ozone generator 16 can be adjusted to provide the desired concentration of ozone. The controller 22 preferably controls the ozone generator 16 so that the concentration of ozone in the ozone containing gas is within a preselected range of acceptable concentrations.

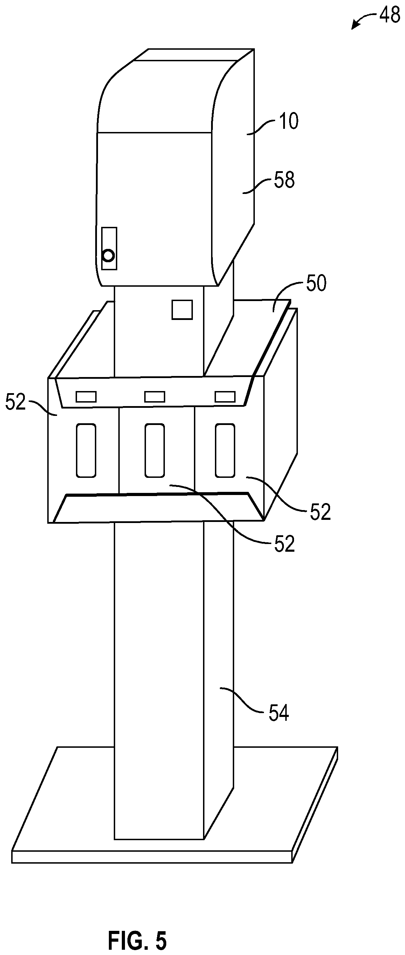

Reference is now made to Figure 5, which depicts a hand hygiene station 48 in accordance with a second embodiment of the present invention. Like numerals represent like components.

The hand hygiene station 48 shown in Figure 5 incorporates a foam dispenser 10 and a glove dispenser 50. The foam dispenser 10 preferably dispenses ozone containing foam 44, similarly to the embodiments shown in Figures 1 to 4. The glove dispenser 50 carries three glove boxes 52 from which gloves 46 can be dispensed. The three glove boxes 52 may, for example, dispense small, medium, and large sized gloves 46, respectively. The glove dispenser 50 and the foam dispenser 10 are mounted on a support post 54, with the foam dispenser 10 positioned directly above the glove dispenser 50. This provides a convenient one-stop location for users to sanitize their hands 28 and put on gloves 46. The hand hygiene station 48 may, for example, be used to coat the user's hands 28 in ozone containing foam 44 prior to placing gloves 46 onto the user's hands 28, as described above with respect to the embodiments shown in Figures 1 to 4. The foam dispenser 10 of the hand hygiene station 48 shown in Figure 5 is optionally identical to the dispenser 10 shown in Figures 1 to 4, and may be used in the same manner as the dispenser 10 shown in Figures 1 to 4 as described above.

The embodiments shown in Figures 1 to 5 therefore provide use of a foam 44 to assist in sliding a hand 28 into a glove 46.

The embodiments shown in Figures 1 to 5 therefore also provide use of an ozone containing foam 44 to treat dermatitis.

The embodiments shown in Figures 1 to 5 therefore also provide use of an ozone containing foam 44 to prevent dermatitis.

The embodiments shown in Figures 1 to 5 therefore also provide a dispenser 10 for dispensing an ozone containing foam 44, the dispenser 10 comprising: an ozone generator 16 that generates an ozone containing gas; a foam generator 24 that receives the ozone containing gas and mixes the ozone containing gas with a foamable liquid to generate the ozone containing foam 44; and a discharge outlet 26 for discharging the ozone containing foam 44; wherein the ozone containing foam 44 is for at least one of: treating dermatitis and preventing dermatitis.

The embodiments shown in Figures 1 to 5 therefore also provide a dispenser 10 for dispensing an ozone containing foam 44, the dispenser 10 comprising: an ozone generator 16 that generates an ozone containing gas; a foam generator 24 that receives the ozone containing gas and mixes the ozone containing gas with a foamable liquid to generate the ozone containing foam 44; a discharge outlet 26 for discharging the ozone containing foam 44; and an ozone adjustor 56 for adjusting an amount of ozone that is dispensed in the ozone containing foam 44 upon activation of the dispenser 10.

The embodiments shown in Figures 1 to 5 therefore also provide a method of placing a glove 46 onto a hand 28 comprising: coating the hand 28 in foam 44; and sliding the hand 28, while the hand 28 is coated in the foam 44, into the glove 46.

The embodiments shown in Figures 1 to 5 therefore also provide a method of placing a glove 46 onto a hand 28 comprising: applying foam 44 into an inner cavity of the glove 46; and sliding the hand 28 into the glove 46.

The embodiments shown in Figures 1 to 5 therefore also provide a method of placing a glove 46 onto a hand 28 comprising: at least one of: coating the hand 28 in foam 44; and applying foam 44 into an inner cavity of the glove 46; and sliding the hand 28 into the glove 46.

The embodiments shown in Figures 1 to 5 therefore also provide a method of treating dermatitis comprising: applying an ozone containing foam 44 to a person's skin.

The embodiments shown in Figures 1 to 5 therefore also provide a method of preventing dermatitis comprising: applying an ozone containing foam 44 to a person's skin.

The embodiments shown in Figures 1 to 5 therefore also provide a method of treating dermatitis on a hand 28 comprising: placing an ozone containing foam 44 into a glove 46; and placing the hand 28 into the glove 46.

The embodiments shown in Figures 1 to 5 therefore also provide a method of preventing dermatitis on a hand 28 comprising: placing an ozone containing foam 44 into a glove 46; and placing the hand 28 into the glove 46.

The embodiments shown in Figures 1 to 5 therefore also provide a dispenser 10 for dispensing an ozone containing foam 44, the dispenser 10 comprising: an ozone generator 16 that generates an ozone containing gas; a foam generator 24 that receives the ozone containing gas and mixes the ozone containing gas with a foamable liquid to generate the ozone containing foam 44; and a discharge outlet 26 for discharging the ozone containing foam 44.

The embodiments shown in Figures 1 to 5 therefore also provide a foam for use in assisting in sliding a hand 28 into a glove 46.

The embodiments shown in Figures 1 to 5 therefore also provide an ozone containing foam 44 for use in treating dermatitis.

The embodiments shown in Figures 1 to 5 therefore also provide an ozone containing foam 44 for use in preventing dermatitis.

The embodiment shown in Figure 5 therefore also provides a hand hygiene station 48 comprising: a foam dispenser 10 for dispensing foam 44; and a glove dispenser 50 for dispensing gloves 46; wherein the foam 44 is for at least one of: assisting in sliding a user's hand 28 into one of the gloves 46; treating dermatitis; and preventing dermatitis.

It will be understood that, although various features of the invention have been described with respect to one or another of the embodiments of the invention, the various features and embodiments of the invention may be combined or used in conjunction with other features and embodiments of the invention as described and illustrated herein.

The present invention is not limited to the particular construction of the foam dispenser 10 as shown and described herein. Rather, the foam dispenser 10 could have any desired structure or combination of features suitable for generating ozone and/or for generating foam 44. The foam dispenser 10 could, for example, incorporate any suitable structures or components as disclosed in United States Patent No. 8,733,596 to Ophardt et al., issued May 27, 2014; U.S. Patent No. 7,303,099 to Ophardt, issued December 4, 2007; U.S. Patent No. 8,272,539 to Ophardt et al., issued September 25, 2012; U.S. Patent No. 8,733,596 to Ophardt et al., issued May 27, 2014; U.S. Patent No. 9,573,152 to Ophardt et al., issued February 21, 2017; and U.S. Patent No. 10,105,018 to Jones et al., issued October 23, 2018, which are incorporated herein by reference.

The invention is also not limited to the particular method of generating foam as described herein, nor to the particular foam generator 24 that has been shown and described. Nor is the invention limited to the exemplary methods of operating the dispenser 10 that are described herein. All other methods of operating the dispenser 10 that would be apparent to a person skilled in the art are included within the scope of the invention.

Although the foamable liquid is preferably a hand cleaning fluid, such as hand sanitizer, other types of foamable liquids could be used instead. The term "foamable liquid" is intended to encompass any flowable substance that is capable of foaming. Although the preferred embodiments have described the ozone containing gas as being ozonated air, this is not necessary. Other types of ozone containing gases could also be used, such as a mixture of oxygen and ozone, a mixture of nitrogen and ozone, or a mixture of nitrogen, oxygen, and ozone.

Any other types of suitable ozone generators 16 could be used instead of the corona discharge chamber 32, including for example generators 16 that use ultraviolet radiation or cold plasma to generate ozone.

All measurements referred to herein are preferably at atmospheric pressure (1 atm) and room temperature (20 °C).

Although this disclosure has described and illustrated certain preferred embodiments of the invention, it is to be understood that the invention is not restricted to these particular embodiments. Rather, the invention includes all embodiments which are functional, electrical, chemical or mechanical equivalents of the specific embodiments and features that have been described and illustrated herein.

## Claims

1. A foam for use in assisting in sliding a hand into a glove.

2. The foam according to claim 1, wherein the foam contains ozone.

3. The foam according to claim 2, wherein the foam has a concentration of ozone that is selected to treat dermatitis on the hand.

4. The foam according to claim 2, wherein the foam has a concentration of ozone that is selected to protect the hand from dermatitis.

5. The foam according to claim 2, wherein the foam has a concentration of ozone that is selected to promote healing of the hand.

6. The foam according to claim 2, wherein the foam has a concentration of ozone that is selected to kill pathogens on the hand.

7. The foam according to any one of claims 1 to 6, wherein the foam contains alcohol for disinfecting the hand.

8. The foam according to any one of claims 1 to 7, wherein the foam comprises bubbles of an ozonated gas in a foamable liquid.

9. The foam according to claim 8, wherein at least 50% of the bubbles remain intact for at least 30 seconds on the hand.

10. The foam according to claim 8 or claim 9, wherein the bubbles have a mean bubble diameter in a range of 50 µm to 600 µm.

11. The foam according to any one of claims 8 to 10, wherein the foam has a ratio of between about 1:5 and about 1:60 of the foamable liquid to the ozonated gas by volume.

12. The foam according to any one of claims 8 to 11, wherein the foam comprises between about 0.5 mL and 10 mL of the foamable liquid and between about 2.5 mL and 100 mL of the ozonated gas.

13. The foam according to any one of claims 8 to 12, wherein the foamable liquid comprises a foaming agent;
wherein the foaming agent comprises a surfactant; and
wherein the foamable liquid comprises about 0.1% to 15% of the surfactant by volume.

14. The foam according to any one of claims 8 to 13, wherein the ozonated gas has between 0.000001% and 0.05% ozone by volume.

15. The foam according to any one of claims 8 to 14, wherein the foamable liquid is a hand disinfectant; and
wherein the ozonated gas comprises ozonated air.
